# EUROPÄISCHE PATENTSCHRIFT

(11) **EP 4 003 504 B1**
(45) Veröffentlichungstag und Bekanntmachung des Hinweises auf die Patenterteilung: **10.01.2024**
(21) Anmeldenummer: 20746186.4
(22) Anmeldetag: 24.07.2020
(51) Int. Cl.: A61N 2/00

(54) **GERÄT ZUR KERNSPINRESONANZTHERAPIE**
APPARATUS FOR NUCLEAR MAGNETIC RESONANCE THERAPY
APPAREIL DE THÉRAPIE PAR RÉSONANCE MAGNÉTIQUE NUCLÉAIRE

(30) Priorität: 24.07.2019 DE 102019119960
(43) Veröffentlichungstag der Anmeldung: 01.06.2022
(73) Patentinhaber: Muntermann, Axel, 35580 Wetzlar (DE)
(72) Erfinder: Muntermann, Axel, 35580 Wetzlar (DE)
(74) Vertreter: Blumbach · Zinngrebe Patentanwälte PartG mbB
(86) Internationale Anmeldenummer: PCT/EP2020/070935
(87) Internationale Veröffentlichungsnummer: WO 2021/013983

(56) Entgegenhaltungen:
- CN-A- 108 144 184
- CN-B- 104 905 902
- CN-B- 105 288 854
- CN-U- 202 776 345
- US-A- 2 273 088
- US-A- 3 060 926
- US-A- 3 404 679
- US-A- 4 144 880
- US-A1- 2003 195 410
- US-A1- 2004 199 070
- US-A1- 2005 080 333
- US-A1- 2013 184 615

## Beschreibung

### Gebiet der Erfindung

Die Erfindung betrifft ein Gerät zur Kernspinresonanztherapie sowie ein Verfahren zu dessen Steuerung. Insbesondere betrifft die Erfindung ein Gerät zur Behandlung degenerativer Veränderungen des Stütz- und Bewegungsapparates, Verletzungen an Bänder, Muskeln, Sehnen und Nerven, krankhaften Veränderungen in den Bandscheiben und den Wirbeln, Sport- und Unfallverletzungen, krankhaften Veränderungen in den knöchernen Strukturen, sowie zur Behandlung von Arthrose-Patienten.

### Hintergrund der Erfindung

Geräte zur Kernspinresonanztherapie sind bekannt. Insbesondere zeigt die Schrift DE 10 2009 060 544 B4 eine Vorrichtung zur Erzeugung von Kernspinresonanzen im zu behandelnden Gewebe. Ferner wird auf das Dokument US 2003/0195410 A1 verwiesen.

Es handelt sich dabei um ein Gerät, welches in einer Behandlungszone ein magnetisches Feld mit homogener Feldstärke erzeugt. Dieses magnetische Feld wird von einem senkrecht hierzu eingestrahlten Wechselfeld überlagert. Durch ein Sweepen des ersten Feldes, also ein leichtes Verändern der Feldstärke, etwa in Form eines Sägezahns, wird erreicht, dass bei jedem Durchlauf ein Erreichen der Resonanzbedingung sichergestellt ist. Es hat sich des Weiteren gezeigt, dass diese sogenannte Sweep-Frequenz, über die ein schneller adiabatischer Durchlauf erreicht wird, mitbestimmend für den Behandlungserfolg ist.

Im Gegensatz zu bildgebenden Magnetresonanztomographen arbeiten Vorrichtungen, welche Kernspinresonanzen zu Heilungszwecken oder kosmetischen Zwecken erzeugen, zumeist mit verhältnismäßig geringen Feldstärken, insbesondere mit Feldstärken, die im mT-Bereich liegen.

Im Gegensatz zu den oben genannten Verfahren der Kernspinresonanztherapie sind andere Verfahren bekannt, bei welche ein zu behandelndes Körperteil innerhalb einer Luftspule gelagert wird, wobei die Luftspule von einem pulsierenden Gleichstrom durchflossen wird. Das Magnetfeld ist damit innerhalb der Spule homogen. Insbesondere liegt kein magnetisches Wechselfeld im Behandlungsbereich vor. Damit handelt es sich bei Geräten dieses Prinzips, welches auch als PST-Verfahren bekannt ist ("PST" = Pulsierende Signaltherapie), um ein anderes Aufgaben- und Anwendungsgebiet als vorliegend, bei welchen die Kernspinresonanztherapie zugrunde gelegt wird.

### Aufgabe der Erfindung

Der Erfindung liegt gegenüber eingangs beschriebenem Stand der Technik die Aufgabe zugrunde, ein Gerät zur Kernspinresonanztherapie weiter zu verbessern.

Es ist insbesondere eine Aufgabe der Erfindung, die Behandlungserfolge, insbesondere bei degenerativen Veränderungen des Stütz- und Bewegungsapparates, Verletzungen an Bänder, Muskeln, Sehnen und Nerven, krankhaften Veränderungen in den Bandscheiben und den Wirbeln, Sport- und Unfallverletzungen, krankhaften Veränderungen in den knöchernen Strukturen, sowie bei der Behandlung von Arthrose-Patienten zu steigern.

### Zusammenfassung der Erfindung

Die Aufgabe der Erfindung wird bereits durch ein Gerät zur Kernspinresonanztherapie, durch ein Modul, insbesondere zur Erzeugung einer zyklisch oder permanent dosierten Streckung eines Benutzers, sowie durch ein Verfahren zur Steuerung eines Geräts zur Kernspinresonanztherapie nach einem der unabhängigen Ansprüche gelöst.

Bevorzugte Ausführungsform und Weiterbildungen der Erfindung sind dem Gegenstand der abhängigen Ansprüche zu entnehmen.

Die Erfindung betrifft ein Gerät zur Kernspinresonanztherapie. Dieses umfasst eine Einrichtung zur Erzeugung eines magnetischen Wechselfeldes sowie eine Einrichtung zur Erzeugung eines quer zum magnetischen Wechselfeld verlaufenden Feldes, insbesondere eines Sweepfeldes.

Insbesondere umfasst das Gerät zur Erzeugung eines magnetischen Wechselfeldes eine Spule. Die Spule kann beispielsweise unterhalb einer Liege angeordnet sein, auf welcher der Benutzer während der Behandlung liegt.

Zur Erzeugung eines Sweepfeldes kann die Vorrichtung insbesondere zwei in Helmholtz-Konfiguration angeordnete Spulen umfassen. Die Spulen werden derart angesteuert, dass das von innen erzeugte magnetische Feld, welches zwischen den Spulen homogen ist, einen Sockelbetrag aufweist, welcher periodisch angehoben und abgesenkt wird. Es versteht sich, dass es bei dieser Ausgestaltung auch denkbar ist, den Sockelbetrag ganz oder teilweise durch eingebaute Permanentmagneten zu erzeugen.

Die Vorrichtung ist insbesondere derart ausgebildet, dass das Sweepfeld in Form eines Sägezahnprofils moduliert wird, wobei während der fallenden Flanken des Sweepfeldes das Wechselfeld angeschaltet ist.

Das erfindungsgemäße Gerät umfasst des Weiteren eine Vorrichtung, die, vorzugsweise dosiert und kontrolliert einstellbar, für eine statische oder impulsierende Streckung eines Benutzers sorgt. Unter einer Vorrichtung zum Strecken eines Benutzers wird eine Einrichtung verstanden, mittels der auf Körperteile des Patienten in Längsrichtung eine insbesondere pulsweise oder permanent gleichbleibende Zugkraft aufgebracht werden kann.

Insbesondere umfasst die Vorrichtung zum Strecken des Benutzers vorzugsweise unterschiedlich geformte Gurte und Befestigungen zum Anlegen sowie zumindest eine Zugvorrichtung für zumindest einen Gurt.

Gemäß einer Ausführungsform der Erfindung ist zumindest ein Gurt mit einem elektrischen oder mechanischen Auslöse- oder Schnellauslösesystem ausgestattet. Der Auslösevorgang kann mit einem akustischen Signal oder einer optischen Meldung dem Fachpersonal oder dem behandelnden Arzt signalisiert werden.

Ab einer bauartbedingten und/oder einstellbaren Maximalkraft wird der Gurt gelöst. So kann eine ungewollte Überstreckung des Benutzers verhindert werden.

Eine weitere Sicherheitsauslösung kann über Sensoren die auf der Haut des Rückens angebracht werden oder über anderen Messeinrichtungen, beispielsweise über eine Messung der Muskelkontraktion durchgeführt werden.

Die Zugvorrichtung ist gemäß einer Ausführungsform der Erfindung elektrisch angetrieben und kann so automatisch in bis zu drei Richtungen oder Ebenen eine, insbesondere pulsierende und/oder eine kontrollierte permanente, Zugkraft vorzugsweise in einem Impulsfrequenzbereich von 0-1 kHz auf den Benutzer ausüben. Der Grad der Verstellung kann über eine akustische oder optische Rückmeldung angezeigt werden.

Da hauptsächlich die Lendenwirbel und die Halswirbel bei Bandscheibenvorfall behandelbar sein sollen, können die einzelnen Liegeflächen diesen Anforderungen anpasst sein. In einer Ausführungsform, die bis zu fünf oder mehr als fünf mögliche einzelnen Liegenplatten haben kann ist in der Mitte der Platte jeweils ein querangeordneter Lagerpunkt oder eine Lagerstange um eine Verstellung der einzelnen Platten der Liege um die Achse des Lagerpunktes mechanisch oder elektrisch zu ermöglich. Dadurch kann gezielt ein Teilbereich der Wirbelsäule, oder insbesondere einzelne betroffene Wirbel in der für den jeweiligen betroffenen Wirbel bestmöglich angepassten Qualität und Intensität der Streckung erreicht werden.

Die Vorrichtung zum Strecken ist insbesondere derart ausgebildet, dass mittels dieser sich gemäß einer Ausführungsform die Wirbelsäule des Patienten in drei Ebenen verschieben und/oder strecken lässt. Hierzu umfasst die Vorrichtung Gurte, welche um den Oberkörper und die Hüfte sowie der Füße und dem Kopfbereich des Benutzers geschnallt werden können. Bestimmte Gurte sind mit einem elektrischen oder mechanischen Auslöse- bzw. Schnellauslösesystem ausgestattet, wodurch eine ungewollte Überstreckung des Benutzers verhindert werden kann.

Bei einer weiteren Ausführungsform der Erfindung ist die Vorrichtung ausgestaltet, um die Beine des Benutzers zu strecken. Hierzu umfasst diese einen Gurt, welcher um die Hüfte gelegt wird sowie Gurte bzw. Schlaufen, welche um die Knöchel des Benutzers gelegt werden können.

Die Zugbewegung kann auch über einen Spindelantrieb mechanisch oder elektrisch in drei Ebenen oder Richtungen ausgeführt werden.

Wirbelverschiebungen oder Muskelverspannungen können hierdurch in das Therapieschema mit einbezogen werden Auch Wirbel- oder Muskelverletzungen wie sie häufig bei Sport- oder Unfallverletzungen stattfinden sind hierdurch behandelbar.

Notwendige gespeicherte therapeutische Bewegungsabläufe während der Behandlung des Bewegungs- und Stützapparates können so problemlos, sicher und wirkungsvoll umgesetzt werden.

Denkbar ist auch, den gesamten Körper des Patienten zu strecken, indem ein Haltesystemgurt im Kopf-, Stirn-Kinnbereich oder ein Gurt um den Oberkörper und zwei weitere Gurte um die Fußgelenke geschnallt werden.

Es versteht sich, dass die Gurte eine beliebige Ausgestaltung haben können und dass insbesondere auch starre Bauteile, welche umgelegt werden können, verwendet werden können.

Der Erfindung liegt die Erkenntnis zugrunde, dass insbesondere bei der Behandlung von degenerativen Veränderungen des Stütz- und Bewegungsapparates, Verletzungen an Bänder, Muskeln, Sehnen und Nerven, krankhaften Veränderungen in den Bandscheiben und den Wirbeln, Sport- und Unfallverletzungen, krankhaften Veränderungen in den knöchernen Strukturen, sowie bei Behandlung von Arthrose-Patienten ein Synergieeffekt bezüglich der Erzeugung von Kernspinresonanzen im zu behandelnden Gewebebereich, insbesondere im zu behandelnden Knochen-, Knorpel-, Muskel- Sehnen- und Nervenbereichen sowie bei Erkrankungen oder Schädigungen in oder an der Wirbelsäule, bei gleichzeitiger mechanischer Stimulation durch permanentes oder pulsierende Schwingungen im Bereich von 0-1 kHz als Strecken in der betroffenen Körperregionen vorhanden ist und zu einer deutlich schnelleren Regeneration der betroffenen Gewebe führt.

Wirbelverschiebungen oder Muskelverspannungen können hierdurch in das Therapieschema mit einbezogen werden Auch Wirbel- oder Muskelverletzungen wie sie häufig bei Sport- oder Unfallverletzungen stattfinden sind hierdurch behandelbar.

Notwendige gespeicherte therapeutische Bewegungsabläufe während der Behandlung des Bewegungs- und Stützapparates können so problemlos, sicher und wirkungsvoll umgesetzt werden.

Neuere Untersuchungen und Studien zeigen, dass durch den Kernspinresonanzeffekt gestörte Zyklen der zirkadianen Rhythmik von Zellen resynchronisiert werden, krankhafte Zustände maßgeblich positiv beeinflusst werden und die ATP sowie der Sauerstoffgehalt in den Zellen hochsignifikant erhöht werden, was den Zellstoffwechsel massiv positiv unterstützt.

Durch eine mechanische Vorspannung durch Zug oder pulsierende Schwingungen während und zusätzlich zu einer Kernspinresonanzbehandlung werden in Gelenken und Knochenbereichen die piezoelektrischen Effekte durch Zug und Druck (Bewegung) hochsignifikant erhöht und somit der Stoffwechselprozess in den betroffenen Geweben deutlich erhöht.

Bei einer Ausführungsform der Erfindung umfasst die Vorrichtung zum Strecken des Benutzers eine über mindestens eine Spindel verstellbare lineargeführte Platte, die in einer Ausführung in drei Ebenen oder drei Richtungen verstellbar sein kann.

Insbesondere umfasst die Vorrichtung zum Strecken des Benutzers mindestens eine lineargeführte verstellbare Platte, welche über eine oder mehrere Spindeln vertikal und/oder horizontal oder in drei Ebenen oder drei Richtungen verstellbar als lineargeführtes verstellbares Kopfteil, Mittelteil, Fußteil oder verschiebbares Vorderteil der Liegefläche ausgebildet ist.

Da hauptsächlich die Lendenwirbel und die Halswirbel bei Bandscheibenvorfall behandelbar sein sollen, können die einzelnen Liegeflächen diesen Anforderungen anpasst sein. In einer Ausführungsform, die bis zu fünf oder mehr als fünf mögliche einzelnen Liegenplatten haben kann, ist in der Mitte der Platte jeweils ein quer angeordneter Lagerpunkt oder eine Lagerstange um eine Verstellung der einzelnen Platten der Liege um die Achse des Lagerpunktes mechanisch oder elektrisch zu ermöglich. Dadurch kann gezielt ein Teilbereich der Wirbelsäule, oder insbesondere einzelne betroffene Wirbel in der für den jeweiligen betroffenen Wirbel bestmöglich angepassten Qualität und Intensität der Streckung erreicht werden.

Durch die Platte kann ein Abschnitt einer Liegefläche bereitgestellt werden. Die Platte ist insbesondere mit Halte-, Klipppunkten oder sonstige Haltemöglichkeiten wie auch Ösen versehen, durch die ein Gurt geführt werden kann.

Es ist insbesondere vorgesehen, dass das Gerät zur Kernspinresonanztherapie mindestens zwei oder mehr Platten die bei einer Ausführungsform vertikal und/oder horizontal oder in drei Ebenen oder drei Richtungen beweglich sind umfasst, von denen mindestens eine Platte über eine oder mehrere Spindeln manuell mechanisch oder mittels elektrisch angesteuerter Motoren lineargeführt verschiebbar ist.

Die Einstellung über die Spindel erfolgt gemäß einer Ausführungsform der Erfindung über mindestens eine Betätigungsvorrichtung am Kopf- oder Fußende oder an einer Seite des Liege- bzw. Traktionsmodules.

Die Zugkraft der Traktion wird über eine Druckdose, Dehnungsmessstreifen oder eine Zugkraftmessung kontrolliert. Die Zug- bzw. Strecklänge kann über eine analoge oder digitale Messskala im Bereich von 0 bis 30 cm oder mehr einstellbar sein.

Gemäß einer weiteren Ausführungsform wird über ein elektronisches Steuerungsgerät, insbesondere mit Chipkartenleser, die Zugkraft, die Verdrehpunkte die Strecklänge, die Verwindung und/oder die Impulsfrequenz gesteuert. Insbesondere können mehrere Stell- oder Schrittmotorenangesteuert werden, Programmzyklen eingespeichert sein. Weiter können Daten betreffend die Verwindung, die Weg- oder Zuglänge und den Traktiongrad, eine Zugkraft der Traktion von 0-60 Kg, eine pulsierende Traktionsbewegung zwischen 0-1 kHz, zeitliche Abläufe, Sicherheitsschutzvorrichtungen und eine Notabschaltung, ergonomische Veränderungen in der Liegefläche und/oder die Höhenveränderungen im Fuß- und Kopfteil in dem Steuerungsgerät abgespeichert sein. Diese können jeweils zu abgespeicherten Patientendaten zugeordnet sein. So kann auf bequeme Weise eine jeweils individuelle Behandlung sichergestellt werden.

Über einen Steuerungscomputer, vorzugsweise mit Display, können die Werte vor und/oder während der Behandlung eingestellt, verändert und kontrolliert werden.

Bei einer bevorzugten Ausführung können diese Werte automatisch vor der Behandlung über Sensoren am dem zu behandelnden Patienten ermittelt und die entsprechenden Daten automatisch über die Geräteelektronik oder die Behandlungschipkarte gespeichert werden.

Die Einstelldaten für die obengenannten Werte sind patientenbezogen in einem elektronischen Speicher oder auf einer Chipkarte gespeichert, was die Reproduzierbarkeit der Einstellungen vor- und während der Behandlung und des Behandlungsablaufes gewährleistet. Hierdurch sind über Sensoren manuelle und elektronische Abschaltungen möglich.

Der Benutzer kann z.B. an einer, insbesondere mittleren Platte, insbesondere mittels mitverstellbaren Fixiervorrichtungen, beispielsweise an der Hüfte fixiert werden. An einer anderen Platte kann der Benutzer entweder an den Beinen oder an den Schultern oder am Kopf fixiert werden.

Weiter kann die erfindungsgemäße Vorrichtung ein Haltegurtsystem umfassen, welches zur Anlage an der Stirn bzw. am Kopfes ausgebildet ist. Hierbei kann es sich um eine Manschette handeln, die die am Kinn und an der Stirn angelegt wird, den Kopf fixiert und Zug über die Halswirbelsäule ermöglicht.

Über eine Verschiebung einer Platte gegenüber anderen Platten kann der Benutzer pulsierend oder permanent ohne oder zusätzlich mit dem Kernspinresonanzsystem gestreckt werden.

Die Platten sind vorzugsweise mit einer Polsterung, insbesondere mit einer Schaumstoffpolsterung, versehen.

Eine oder mehrere Verbindungsschutzelemente unter oder zwischen den Platten, z.B. in Form von Gummi- oder Kunststofflippen oder Faltenbälgen, können dafür sorgen, dass keine Verschmutzungen durch die Spalte zwischen den Platten in das darunterliegende Behandlungsgerät fallen können.

Die verschiebbaren Platten sind insbesondere über einen oder mehrere stirnseitige Antriebe in der Lage und Stellung veränderbar.

Insbesondere ist ein Antrieb mit einer Gewindespindel vorgesehen, über die eine Platte entweder manuell oder mittels eines Elektromotors, insbesondere permanent oder pulsierend, linear in verschiedenen Ebenen oder Richtungen veränder- und/oder verschiebbar ist.

Der Antrieb, insbesondere eine Gewindespindel oder eine Stange zum Bewegen einer verschiebbaren Platte ist gemäß einer Ausführungsform der Erfindung zumindest abschnittsweise unter einer anderen, nicht verschiebbaren Platte angeordnet.

Es ist insbesondere vorgesehen, dass der Antrieb stirnseitig, also vorne oder hinten oder an einer der Längsseiten an dem Gerät zur Kernspinresonanztherapie angeordnet ist, wobei eine mittlere oder eine oder mehrere Endplatten über einen unter einer nicht verschiebbaren Platte hindurchlaufenden Mechanismus verschiebbar ist.

So kann in vorteilhafter Weise eine Bedieneinrichtung bereitgestellt werden, welche vorne oder hinten an dem Gerät zur Kernspinresonanztherapie angeordnet ist, also der Seite, von welcher das Strecken des Benutzers und in der Regel auch das Anlegen der Gurte erfolgt.

Bei einer Weiterbildung der Erfindung ist die Vorrichtung zum Strecken des Benutzers als auf ein Gehäuse aufsetzbares Modul ausgebildet.

Gemäß einer weiteren Ausführungsform ist die Platte des Fußteils mit einem Luft-, Kunststoff-, Gummisack oder Faltenbalg verbunden, der elektrisch- oder mechanisch mit Luft- oder Flüssigkeit gefüllt werden kann, um das Fußteil zu dem Rest der Liegefläche, insbesondere zwischen 1 und 60 cm, vertikal anzuheben.

Eine entsprechende mechanische, elektrische oder elektronische Steuerungseinheit kann hierfür vorgesehen sein.

Diese Funktion kann auch über eine zum Beispiel mechanischen Scherenhebebühne, Teleskopführung oder ähnliches Hebesystem erreicht werden, die das Fußteil gezielt anheben kann.

Diese Vorrichtung sorgt, zum Beispiel bei einer Bandscheibenerkrankung oder -verletzung, für eine Entlastung der Wirbelsäule.

Hierdurch werden bei der Behandlung keine weiteren Unterstützungsteile wie z.B. Würfel oder ähnliches benötigt, der Liegekomfort des Anwenders hierdurch deutlich erhöht.

Gemäß einer weiteren Ausführungsform kann das Kopfteil über einen Luft- oder Gummisack oder Faltenbalg, der manuell oder über eine mechanische oder elektrisch Luft- oder Flüssigkeitspumpe gefüllt werden kann, angehoben werden. So kann eine Unterstützung des Kopfbereiches bereitgestellt werden.

Die Luftmenge für die Kopf- oder Fußanhebung kann über ein elektronisches oder mechanisches Manometer oder sonstiges Druckmesssystem kontrolliert- und überwacht werden.

Gemäß einer Ausführungsform der Erfindung ist ein Modul vorgesehen, welches einen Rahmen umfasst, an welchem zumindest eine verschiebbare Platte angeordnet ist.

Die Vorrichtung zum Strecken des Benutzers kann so als Nachrüstset bereitgestellt werden, welches gegen das Polster der Vorrichtung ausgetauscht wird oder welches unter das Polster der Vorrichtung gesetzt wird.

Insbesondere ist die Vorrichtung zum Strecken des Benutzers derart ausgebildet, dass insbesondere über eine Zugvorrichtung eine periodisch wechselnde Kraft erzeugbar ist.

Durch eine periodisch wechselnde Kraft, welche beispielsweise mittels eines elektrischen Antriebs und entsprechender Steuerung erzeugt werden kann, wird eine Schwingung auf die zu behandelnden Körperregionen aufgeprägt und der Knorpel bzw. das Gewebe wird während der Behandlung mit Kernspinresonanzen derart stimuliert, dass die Teilungsrate und somit die Bildung von Körperzellen in den verschiedensten zu behandelten Gewebearten wesentlich verbessert wird.

Die Frequenz der periodisch wechselnden Kraft beträgt insbesondere 0,01 bis 1 kHz.

Bei einer Weiterbildung der Erfindung umfasst die Vorrichtung zum Strecken des Benutzers einen Aktuator zum Aufbringen einer Schwingung auf die Zugvorrichtung.

Diese Ausführungsform der Erfindung betrifft mithin ein Gerät, welches eine Zugvorrichtung zum Erzeugen einer Kraft sowie einen zusätzlichen Aktuator zum Aufbringen einer Schwingung umfasst, welche die von der Zugvorrichtung aufgebrachte Kraft überlagern.

Zur Erzeugung der Traktion sind mechanische Exzenterscheiben mit Hubstangen möglich.

In dieser Ausführungsform ist die Traktionsgeschwindigkeit elektrisch, elektronisch oder mechanisch regelbar

Es sind insbesondere Aktuatoren vorgesehen, welche ein Gewicht umfassen, das magnetisch in Schwingung versetzt werden kann. Insbesondere können auch Tauchspulenaktuatoren verwendet werden.

Zwar ist auch über eine elektrisch angetriebene Zugvorrichtung eine periodisch wechselnde Kraft erzeugbar. Über einen Aktuator kann aber auf einfachere Weise auch eine recht hochfrequente Kraftänderung aufgebracht werden, ohne dass die Gefahr besteht, dass durch Dämpfung innerhalb der Zugvorrichtung diese größtenteils abgeschwächt wird.

Dies gilt insbesondere dann, wenn die Aktuatoren relativ nah an den Gurten für den Benutzer angebracht sind oder direkt an diesen angebracht oder in die Gurte integriert sind. Vorzugsweise beträgt der Abstand des Aktuators von einem Gurt weniger als 50 cm.

Der Aktuator ist insbesondere ausgebildet mit einer Frequenz zwischen 0,1 und 1 kHz, bevorzugt zwischen 1 und 200 Hz, zu schwingen.

Bei einer Weiterbildung der Erfindung umfasst das Gerät eine Steuereinrichtung für die Einrichtung zur Erzeugung des magnetischen Wechselfeldes und für das Sweepfeld, welche gleichzeitig auch zur Steuerung der Vorrichtung zum Strecken des Benutzers ausgebildet ist.

Sämtliche Behandlungsparameter können so komfortabel über eine einzige Steuerungseinrichtung eingegeben und ggf. automatisiert, z.B. über Chipkarten oder Speichermedien, ausgeführt werden.

Weiter wirkt sich möglicherweise positiv auf den Behandlungserfolg aus, wenn die Frequenz, mit welcher die Kraft zur Streckung des Benutzers moduliert wird, auf die Frequenz des Sweepfeldes synchronisiert wird. Hierunter wird auch verstanden, dass die Frequenz, mit welcher die Kraft zum Strecken moduliert wird, ein ganzzahliges Vielfaches oder einen Bruchteil der Frequenz der Modulation des Sweepfeldes beträgt.

Es ist aber insbesondere auch vorgesehen, dass die Kraft zur Streckung des Benutzers derart moduliert wird, dass immer in den fallenden Flanken des Sweepfeldes diese Kraft ansteigt oder fällt.

Bei einer bevorzugten Ausführungsform der Erfindung ist das Gerät zur Kernspinresonanztherapie als Liege ausgebildet, wobei seitlich an der Liege zwei Seitenteile angeordnet sind, welche jeweils eine Spule umfassen. Diese seitlich angeordneten Spulen sind in Helmholtz-Konfiguration angeordnet und dienen der Erzeugung des Sweepfeldes. Mittels zumindest einer Spule unter der Liege wird quer zum Sweepfeld ein magnetisches Wechselfeld eingestrahlt.

Die Erfindung betrifft des Weiteren ein Modul zum Strecken eines Benutzers, welches insbesondere für ein Gerät zur Kernspinresonanztherapie, wie es vorstehend beschrieben wurde, verwendbar ist.

Das Modul ist insbesondere als Nachrüstsatz ausgebildet, welcher auf ein Gehäuse einer Vorrichtung zur Behandlung mit Kernspinresonanzen aufsetzbar ist.

Das Modul umfasst einen auf ein Gehäuse aufsetzbaren Rahmen mit zumindest zwei Platten, bevorzugt mit drei Platten, die eine Liegefläche bilden, wobei eine der Platten gegenüber der oder den anderen Platten mittels eines Antriebs verschiebbar ist.

In weiterer Ausgestaltung umfasst das Modul drei oder mehr als drei, insbesondere auch fünf Platten, wobei zumindest eine mittlere oder vordere oder jede der Platten verschiebbar ist, insbesondere auf Schienen, die am Rahmen angeordnet sind, insbesondere mittels eines Antriebs, der eine zumindest abschnittsweise unter einer nicht verschiebbaren Platte laufende Spindel oder Stange umfasst.

An der verschiebbaren Platte sowie an zumindest einer der anderen Platten kann jeweils ein Gurt oder mehrere angebracht werden.

Durch eine Verschiebung der einen Platte zu den anderen Platten kann sodann der Benutzer dosiert gestreckt werden.

Bei einer Ausführungsform umfasst das Modul mindestens zwei Platten, wobei eine, insbesondere eine mittlere oder mehrere Platten verschiebbar sind.

Gemäß einer anderen Ausführungsform ist eine oder mehrere Endplatten verschiebbar.

Insbesondere bei der Ausführungsform mit einer mittleren verschiebbaren Platte kann sich ein Teil des Antriebs, insbesondere eine Kraftmesseinrichtung unter einer nicht verschiebbaren Platte befinden. So wird eine kompakte Ausgestaltung bereitgestellt.

Die verschiebbare Platte oder die Platten sind insbesondere auf Schienen, welche am Rahmen angeordnet sind, verschiebbar.

Als Antrieb wird insbesondere ein Spindelantrieb verwendet.

Die Länge der Streckung sowie die Impuls- und/oder Zugkräfte sind vorzugsweise über geeignete Mess- und Kontrolleinrichtungen überwach- und prüfbar, eine Statusanzeige akustisch und/oder optisch ist möglich und bei bestimmten Ausführungsformen vorgesehen.

Die Erfindung betrifft des Weiteren ein Verfahren zur Steuerung eines Gerätes zur Kernspinresonanztherapie, insbesondere eines vorstehend beschriebenen Gerätes.

Mittels einer Steuereinrichtung werden ein magnetisches Wechselfeld und ein quer dazu stehendes Sweepfeld erzeugt. Mittels dieser magnetischen Felder werden Kernspinresonanzen im Gewebe eines Benutzers erzeugt. Gleichzeitig wird der Benutzer während der Erzeugung von Kernspinresonanzen mittels einer von der Steuereinrichtung angesteuerten Zugvorrichtung gestreckt.

Die Steuereinrichtung ist insbesondere programmierbar hinsichtlich der Dauer der Behandlung, der Frequenz der Modulation des Sweepfeldes sowie optional einer Frequenz der Modulation der von der Vorrichtung zum Strecken des Benutzers auch ausgeübten Kraft.

Insbesondere ist vorgesehen, dass auch die Behandlung automatisch beendet wird. Auch das Abschalten der Zugvorrichtung erfolgt vorzugsweise automatisch.

Es ist weiterhin insbesondere vorgesehen, dass die Frequenz der Modulation der Kraft, mittels der der Benutzer gestreckt wird, auf die Modulationsfrequenz des Sweepfeldes abgestimmt wird, etwa in Form eines ganzzahligen Bruchteils oder Vielfachen.

### Kurzbeschreibung der Zeichnungen

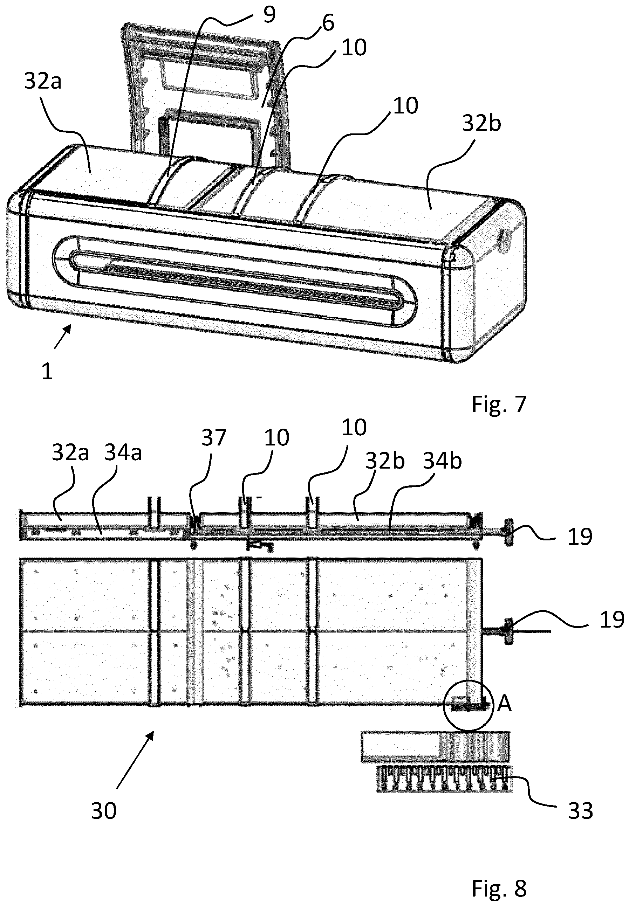

Die Erfindung soll im Folgenden bezugnehmend auf die Zeichnungen Fig. 1 bis Fig. 10 anhand von Ausführungsbeispielen näher erläutert werden.
Fig. 1 zeigt eine perspektivische Ansicht einer ersten Ausführungsform eines Gerätes zur Kernspinresonanztherapie.
Fig. 2 zeigt eine weitere perspektivische Ansicht einer Ausführungsvariante, welche zusätzlich Aktoren zum Aufprägen einer Schwingung auf die Zugvorrichtung umfasst.
Fig. 3 ist eine Detaildarstellung der Fig. 2.

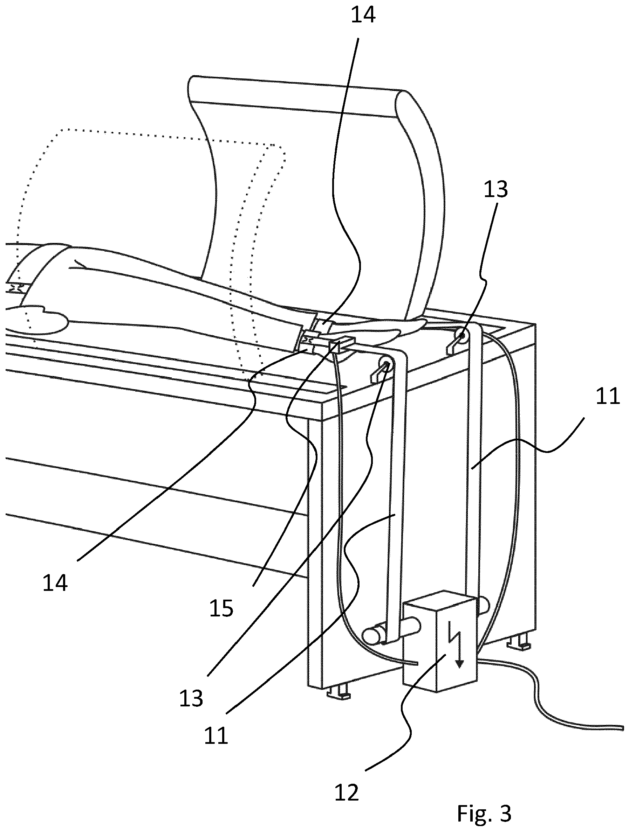

Fig. 3 bis Fig. 6 zeigen eine weitere Ausführungsform der Erfindung, bei welcher die Vorrichtung zum Strecken des Benutzers als nachrüstbares Modul ausgebildet ist.

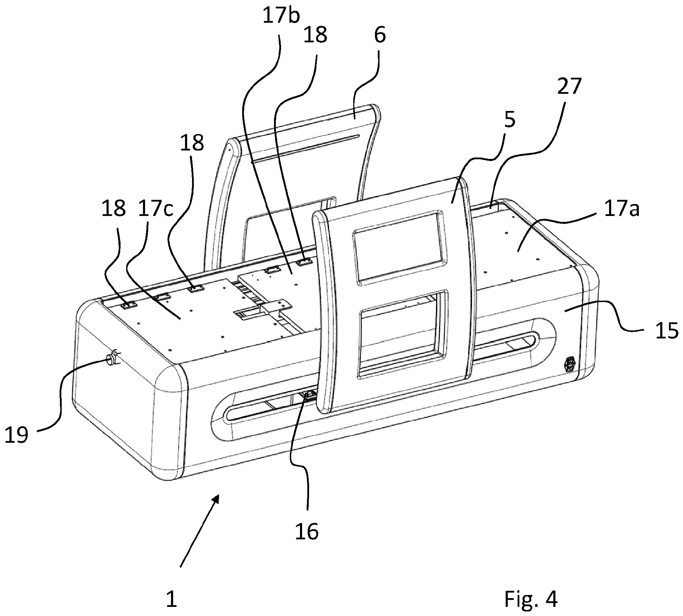

Fig. 4 ist eine perspektivische Ansicht des mit dem Modul bestückten Gerätes zur Kernspinresonanztherapie.

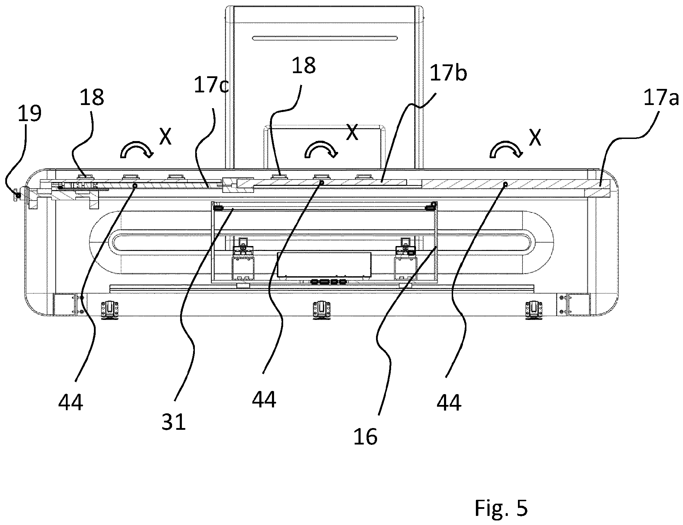

Fig. 5 ist eine Schnittansicht entlang der Haupterstreckungsrichtung.

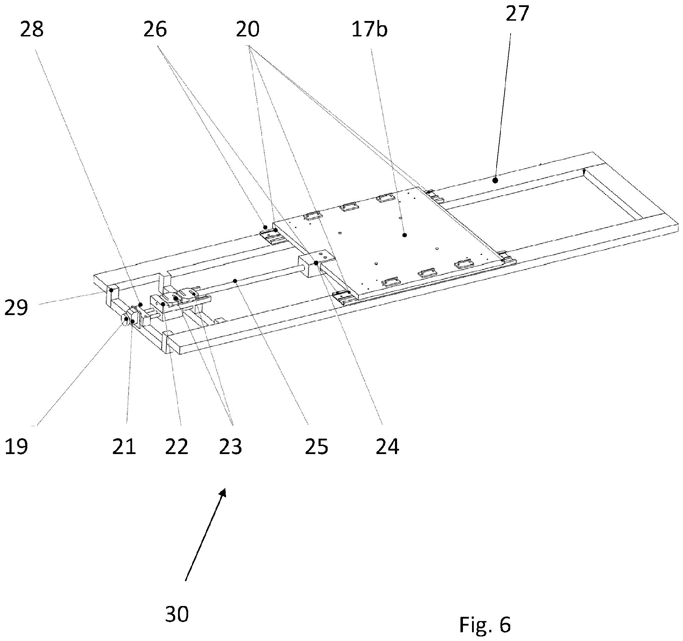

Fig. 6 zeigt in einer perspektivischen Ansicht das nachrüstbare Modul.
   Bezugnehmend auf Fig. 7 bis Fig. 10 soll eine weitere Ausführungsform eines erfindungsgemäßen Gerätes zur Kernspinresonanztherapie näher erläutert werden.
Fig. 7 ist eine perspektivische Ansicht.
Fig. 8 ist eine Draufsicht sowie eine Seitenansicht einschließlich einer Detaildarstellung einer Skala.

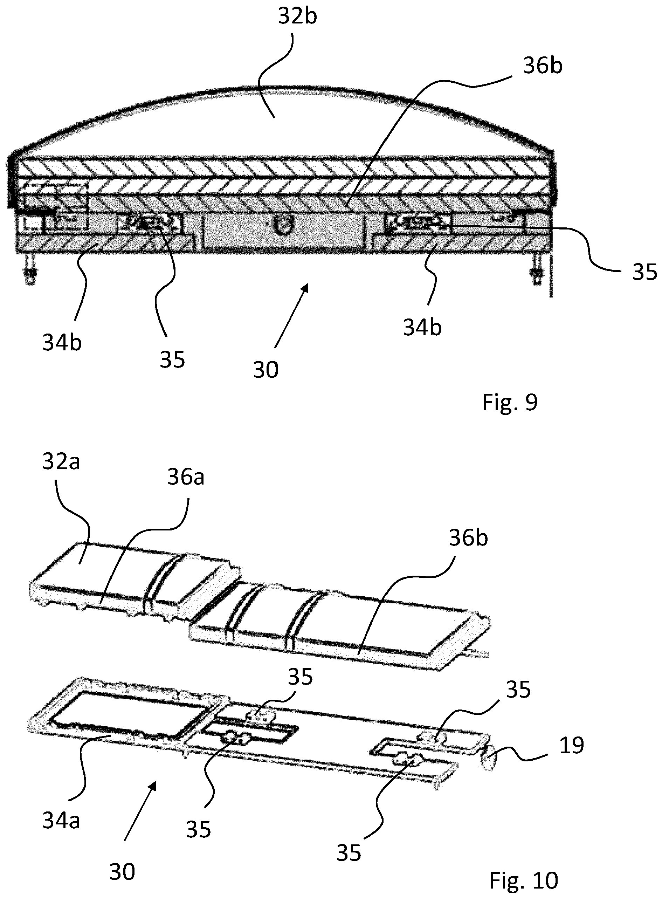

Fig. 9 ist eine axiale Schnittansicht des Gerätes.
Fig. 10 zeigt in einer Explosionsdarstellung die Bestandteile des Moduls, welches für das in Fig. 7 bis Fig. 10 dargestellte Gerät zum Strecken des Benutzers verwendet wird.

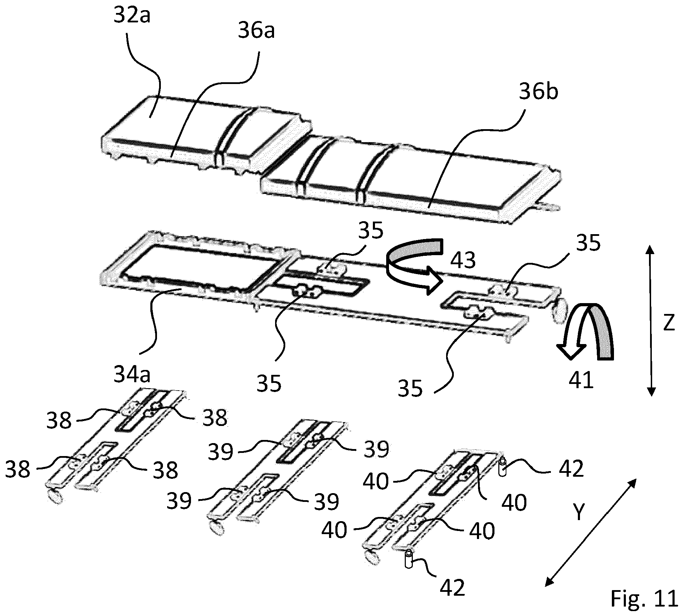

Fig. 11 zeigt in einer weiteren Explosionsdarstellung die Bestandteile des Moduls, welches für das in Fig. 7 bis Fig. 10 dargestellte Gerät zum Strecken des Benutzers verwendet wird, bei welchem jedoch das Strecken in drei Ebenen oder Richtungen einstellbar ist.

### Detaillierte Beschreibung der Zeichnungen

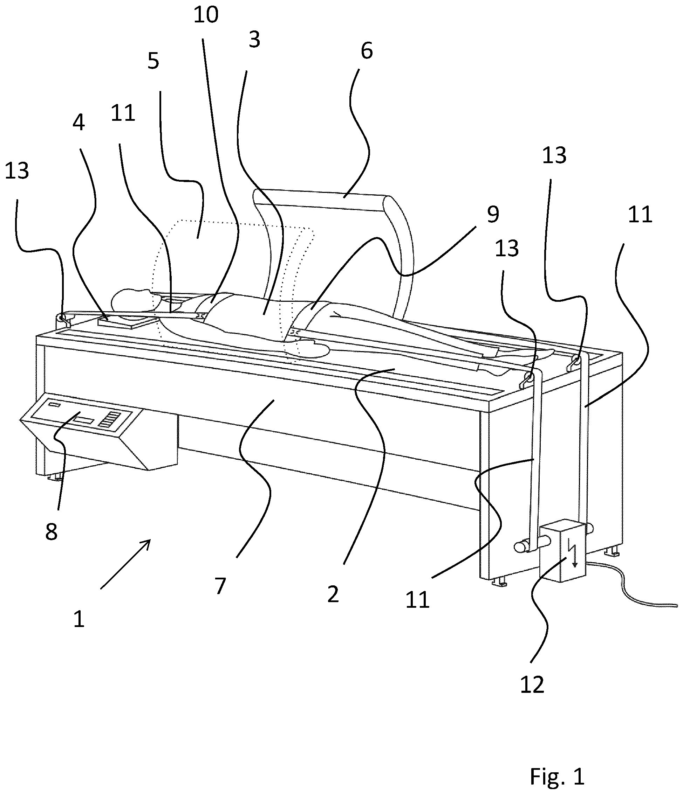

Fig. 1 zeigt eine perspektivische schematische Darstellung eines Gerätes zur Kernspinresonanztherapie 1.

Das Gerät zur Kernspinresonanztherapie 1 umfasst eine Liege 2, auf welcher sich der Benutzer 3 hinlegen und seinen Kopf auf der Kopfstütze 4 absetzen kann.

Das Gerät umfasst die beiden, in dieser Ausführungsform gekrümmt ausgestalteten Seitenteile 5 und 6, welche randseitig an der Liege 2 angeordnet sind.

Liege 2 und Seitenteile 5, 6 sind auf einem Träger 7 angeordnet, an welchem auch eine Steuereinrichtung 8 angeordnet ist.

In den Seitenteilen 5 und 6 befindet sich jeweils eine Spule. Mittels dieser Spulen wird das Sweepfeld erzeugt. Die Spulen (nicht dargestellt) werden von der Steuereinrichtung 8 angesteuert.

Eine weitere Spule (nicht dargestellt) ist unterhalb der Liege 2 angeordnet und dient der Erzeugung eines magnetischen Wechselfeldes, welches sich senkrecht mit dem Sweepfeld überlagert.

Das Gerät zur Kernspinresonanztherapie umfasst des Weiteren eine Vorrichtung zum Strecken des Benutzers 3. Diese umfasst einen Gurt 9, welcher um die Hüfte des Benutzers 3 gelegt ist sowie einen Gurt 10, welcher unterhalb der Achseln um den Oberkörper des Benutzers gelegt ist.

Der Gurt 9 ist über Verbindungsgurte 11, welche am Ende der Liege über Umlenkrollen 13 laufen, mit einer motorisch angetriebenen Zugvorrichtung 12 verbunden.

Es versteht sich, dass die Verbindungsgurte 11 eine beliebige Ausgestaltung haben können und dass statt Gurten auch Seile oder Ketten denkbar sind.

Die Zugvorrichtung 12 wird ebenfalls von der Steuereinrichtung 8 angesteuert. Mittels der Zugvorrichtung 12 können die Gurte 9 und 10 auseinandergezogen werden und üben so eine Kraft auf die Wirbelsäule des Benutzers 3 aus.

Bei dieser Ausführungsform der Erfindung ist auch der Gurt 10 über Verbindungsgurte 11, welche Über Umlenkrollen 13 laufen, mit einer weiteren Zugvorrichtung (nicht dargestellt) verbunden. Es versteht sich aber, dass zum Aufbringen einer Kraft eine Zugvorrichtung ausreichend ist.

Die Verwendung der Umlenkrollen hat den Vorteil, dass die Zugvorrichtung 12 nicht auf- oder direkt angrenzend zur Liegefläche montiert werden muss.

Insbesondere bei einer kompakt ausgestalteten Zugvorrichtung ist aber auch denkbar, auf die Umlenkrollen 13 zu verzichten und die Zugvorrichtung direkt am Ende der Liege 2 anzuordnen.

Die Steuerungsvorrichtung 8 umfasst eine Bedieneroberfläche oder einen Chipkarteneinschub, worüber eine Behandlung automatisch gestartet und ausgeführt werden kann. Dabei steuert die Steuereinrichtung 8 sowohl die in den Seitenteilen 5 und 6 angeordneten Sweepspulen sowie die Spule zur Erzeugung des magnetischen Wechselfeldes als auch die Zugvorrichtung 12.

So kann insbesondere mittels der Zugvorrichtung 12 eine periodisch wechselnde Kraft erzeugt werden. Diese Kraft kann insbesondere auf die Modulationsfrequenz des Sweepfeldes synchronisiert werden.

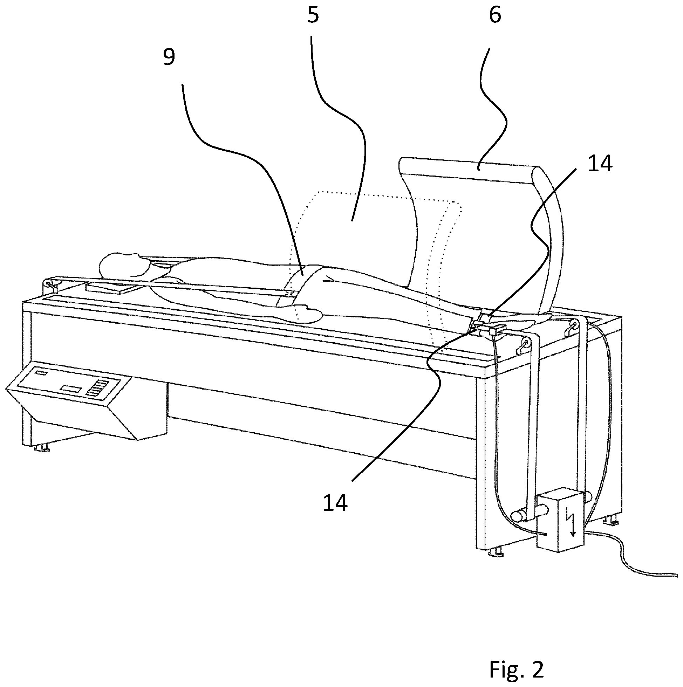

Fig. 2 zeigt eine Ausführungsvariante, welche bis auf die nachstehend beschriebenen Unterschiede der Fig. 1 entspricht.

Im Unterschied zu Fig. 1 werden in der Darstellung in Fig. 2 die Beine des Benutzers gestreckt. Hierzu umfasst die Vorrichtung zum Strecken den Gurt 9, welcher um die Hüfte geschnallt wird sowie die Gurte 14, welche um die Fußgelenke des Benutzers geschnallt sind.

Die Seitenteile 5 und 6 sind nunmehr im Bereich der Beine des Benutzers angeordnet.

Denkbar ist insbesondere, dass die Seitenteile 5 und 6 verschiebbar gegenüber der Liege ausgestaltet sind. So kann die Vorrichtung angepasst werden, um Beine oder Wirbelsäule zu behandeln.

Es versteht sich, dass auch eine Wechselspule unterhalb der Beine angeordnet sein muss. Hierzu ist es denkbar, entweder auch die Wechselspule verschiebbar auszugestalten oder mehrere Wechselspulen unterhalb der Liege vorzusehen.

Fig. 3 zeigt eine Detaildarstellung der Fig. 2, in welcher nunmehr zu erkennen ist, dass die Verbindungsgurte 11 Aktuatoren 15 umfassen, welche direkt angrenzend an den Gurten 14, die um die Fußgelenke geschnallt sind, angeordnet sind.

Denkbar ist auch, die Aktuatoren 15 in die Gurte 14 zu integrieren (nicht dargestellt).

Die Aktuatoren 15 sind mithin gesehen von der Zugvorrichtung 12 nach den Umlenkrollen und somit nah am Benutzer angeordnet.

Die Aktuatoren 15 arbeiten beispielsweise auf dem elektromagnetischen Prinzip, sind beispielsweise als Tauchspulenaktuatoren angeordnet. Durch periodisches Hin- und Herschwingen eines Gewichts kann über die Aktuatoren 15 eine periodisch wechselnde Kraft erzeugt werden, welche sich mit der Kraft der motorischen Zugvorrichtung 12 überlagert.

Die Aktuatoren 15 sind hierzu in dieser Ausführungsform über die Zugvorrichtung 12 mit der Steuereinrichtung (8 in Fig. 1) verbunden. Über die Aktuatoren können insbesondere hochfrequentere Schwingungen zur Modulation der Kraft erzeugt werden. Dies wäre über die Zugvorrichtung 12, welche motorisch betrieben wird, zum einen schwieriger und es bestünde insbesondere bei hochfrequenteren Schwingungen die Gefahr, dass durch Dämpfungseffekte in den Verbindungsgurten 11 die Kraftmodulation nicht mehr an den Gurten 14 ankommt.

Fig. 4 zeigt in einer perspektivischen Ansicht ein Gerät 1 zur Kernspinresonanztherapie 1. Die Liegefläche des Geräts zur Kernspinresonanztherapie 1 wird in diesem Ausführungsbeispiel durch die Platten 17a bis 17c gebildet.

Die mittlere Platte 17b ist gegenüber den anderen Platten 17a, 17c verschiebbar ausgebildet.

Es versteht sich, dass die Platten 17a bis 17c eine hier nicht dargestellte Polsterung umfassen können.

In einer in Figur 5 gezeigten Ausführungsform, welche nicht nur die explizit dargestellten Platten 17a bis 17c umfasst, sondern bis zu fünf oder mehr als fünf mögliche einzelnen Liegenplatten, somit noch zwei oder mehrere weitere, jedoch in Figur 5 um der Einfachheit Willen nicht dargestellte Lagerplatten umfassen kann, ist jeweils ein quer, insbesondere senkrecht zur jeweiligen Längsrichtung der Platte 17a bis 17c angeordneter Lagerpunkt 44 oder eine Lagerstange 44 vorgesehen, um eine Verstellung, insbesondere Verkippung der einzelnen Platten der Liege um die jeweilige Achse des Lagerpunktes 44 somit in positiver oder negativer Richtung des in Figur 5 dargestellten Pfeils X mechanisch oder elektrisch zu ermöglichen. Dadurch kann gezielt ein Teilbereich der Wirbelsäule, und können insbesondere einzelne betroffene Wirbel in der für den jeweiligen betroffenen Wirbel bestmöglich angepassten Qualität und Intensität der Streckung erreicht werden.

Um derartige Verkippungen zu ermöglichen, können die unter dem Modul 30 oder der Liege 1 liegenden Baugruppen etwas tiefer als in den Zeichnungen dargestellt angeordnet sein und müssen auf diese Weise der Verkippung nicht im Wege stehen.

Wegen des Sweep-Feldes ist dies für die sich insgesamt ergebende Feldanordnung sowie den angestrebten Behandlungserfolg unkritisch.

Bei dieser Ausführungsform der Erfindung umfasst das Gerät zur Kernspinresonanztherapie 1 die Seitenteile 5 und 6, welche an einem verschiebbaren Schlitten 16 angeordnet sind.

In den Seitenteilen 5, 6 befinden sich wiederum Spulen, über die ein sich quer über die Liegefläche erstreckendes Magnetfeld erzeugbar ist.

Eine weitere Spule (nicht dargestellt) befindet sich an dem Schlitten 16 unterhalb der Liegefläche. Über diese Spule wird ein Wechselfeld in die Behandlungszone eingestrahlt, welches quer zu dem Feld verläuft, welches durch die in den Seitenteilen 5, 6 vorhandenen Spulen erzeugt wird.

Um den Benutzer zu strecken, sind die Platten 17b und 17c mit Ösen 18 versehen, die dem Durchführen von Gurten dienen.

Bei einer Ausführungsform der Erfindung ist auch vorgesehen, dass eine der Platten mit einer Erhöhung, insbesondere einer keilförmigen Erhöhung (nicht dargestellt) versehen wird, an welcher der Benutzer die Beine hochlegen kann.

Durch Betätigen eines stirnseitig angeordneten Antriebs 19 kann die mittlere Platte 17b horizontal verschoben werden, um so den Benutzer zu strecken.

Der Antrieb ist vorzugsweise derart ausgebildet, dass die Strecke zum Verschieben auf weniger als 50, vorzugsweise auf weniger als 5 cm, begrenzt ist, um eine Sicherheit des Geräts 1 auch bei Benutzerfehlern zu gewährleisten.

Die Einrichtung zum Strecken des Benutzers ist in diesem Ausführungsbeispiel als nachrüstbares Modul ausgebildet, welches einen Rahmen 27 mit den Platten 17a bis 17c umfasst, welcher auf das Gehäuse 15 des Geräts 1 zur Kernspinresonanztherapie aufgesetzt werden kann.

Fig. 5 ist eine Schnittansicht entlang der Haupterstreckungsrichtung des in Fig. 4 dargestellten Geräts 1 zur Kernspinresonanztherapie.

Zu erkennen sind insbesondere die drei hintereinander angeordneten Platten 17a bis 17c, von denen die mittlere Platte 17b horizontal verschiebbar ist.

Der ebenfalls verschiebbare Schlitten mit den Seitenteilen und einer Spule 31 zur Erzeugung eines magnetischen Wechselfeldes ist ebenfalls verschiebbar.

Über den stirnseitig angeordneten Antrieb 19 kann die Platte 17b hin und her bewegt werden.

Fig. 6 ist eine perspektivische Ansicht des Moduls 30, welches einen Rahmen 27 umfasst, der auf ein Gehäuse aufgesetzt werden kann.

Die vordere und hintere Platte (17a und 17c in Fig. 5) sind in dieser Ansicht ausgeblendet.

Die mittlere Platte 17b umfasst Führungen 20, über welche die mittlere Platte 17b auf Schienen 26 entlang des Rahmens 27 verschiebbar ist.

Die Schienen 26 sind auf dem Rahmen angebracht.

Um die mittlere Platte 17b zu verschieben, ist an einer Verstrebung 29 des Rahmens der Antrieb 19 angebracht.

Der Antrieb 19 umfasst eine Spindel 28, mittels der ein Schlitten 22 verschiebbar ist.

Der Schlitten 22, welcher unter der vorderen Platte (17c in Fig. 5) sitzt, ist über eine Stange 25 mittels eines Verbinders 24 mit der mittleren Platte 17 gekoppelt.

Über den Antrieb 19 wird also der Schlitten 22 bewegt und bewegt seinerseits über die Stange 25 die verschiebbare Platte 17b.

Des Weiteren umfasst das Modul 30 eine Kraftmesseinrichtung.

In diesem Ausführungsbeispiel sind Kraftsensoren 23, insbesondere ausgebildet als S-förmige Wägezellen, auf dem Schlitten 22 angeordnet.

Die Messeinrichtung ist also vorzugsweise ebenfalls unter einer Platte (17c) angeordnet.

Die vom Kraftsensor 23 gemessene Kraft wird auf einer Anzeige 21 angezeigt.

Die Anzeige 21 kann als Kraftanzeige und/oder Positionsanzeige ausgebildet sein.

Über die Anzeige 21 kann insbesondere der Hub, um den die mittlere Platte 17b verschoben wird, bestimmt werden.

Denkbar ist auch, die Anzeige 21 mit einer Bedieneinrichtung zu versehen, über die beispielsweise ein Programm gestartet werden kann, welches über einen elektromotorisch ausgeführten Antrieb die Platte 17b periodisch bewegt.

Eine Steuereinrichtung (nicht dargestellt) für den Antrieb kann insbesondere auch mit dem Kraftsensor 23 zusammenwirken, etwa, dass der Hub in Abhängigkeit von der Kraft gesteuert wird.

Fig. 7 zeigt in einer perspektivischen Ansicht ein Gerät 1 zur Kernspinresonanztherapie, welches entsprechend den zuvor dargestellten Ausführungsbeispielen Seitenteile 6 mit Spulen umfasst.

Das gegenüberliegende Seitenteil ist in dieser Ansicht zur besseren Übersicht ausgeblendet.

Das Gerät 1 zur Kernspinresonanztherapie ist ebenfalls modular aufgebaut und umfasst eine Liegefläche, die aus den Polstern 32a und 32b zusammengesetzt ist.

Die Liegefläche ist also in zwei Bereiche unterteilt, welche zum Strecken des Benutzers gegeneinander verschoben werden können.

Ein Gurt 9 liegt über dem Polster 32a.

Zwei voneinander beabstandete Gurte 10 liegen über dem Polster 32b.

Die Polster 32a und 32b sind Teil eines aufsetzbaren Moduls 30, welches in einer Draufsicht und einer Seitenansicht in Fig. 8 dargestellt ist.

Das Modul 30 umfasst die Schienen 34a und 34b, auf welchen jeweils die Polster 32a, 32b angeordnet sind.

Über einen Antrieb 19, in diesem Ausführungsbeispiel als Handantrieb ausgebildet, kann die Schiene 34b und damit das Polster 32b, zusammen mit den Gurten 10 gegenüber dem Polster 32a verschoben werden.

Der Antrieb 19 kann in diesem Ausführungsbeispiel stirnseitig an der Schmalseite des Moduls 30 angebracht sein.

Wie in der Detaildarstellung des Bereiches A unten rechts zu erkennen, ist randseitig eine Skala 33 angebracht, auf welcher die Verschiebung des Schlittens abgelesen werden kann, welcher auf den Schienen 34b gelagert ist.

Über die Skala 33 kann so die Streckung des Benutzers abgelesen werden.

Fig. 9 ist eine axiale Schnittansicht des Moduls 30.

Auf den Schienen 34b ist über die Lager 35 die Platte 36b linear verfahrbar gelagert.

Die Platte 36b kann so mit dem Polster 32b verschoben werden.

Fig. 10 ist eine Explosionsdarstellung der Komponenten des Moduls.

Die Schienen 34a sind Teil eines Rahmens, auf welches ein Kopfteil mit dem Polster 32a und der Platte 36a aufgesetzt werden kann.

Dieses Kopfteil ist in diesem Ausführungsbeispiel fest mit dem restlichen Gerät zur Kernspinresonanztherapie verbunden, wird also nicht zum Strecken des Benutzers verschoben.

Die Platte 36b ist dagegen auf den Lagern 35 axial entlang der Haupterstreckungsrichtung linear verfahrbar, was in diesem Ausführungsbeispiel über den Spindelantrieb 19 realisiert ist.

Die beiden gegeneinander verschiebbaren Teile mit den Polstern 32a und 32b sind, wie in Fig. 8 dargestellt, über die Verbindung 37 miteinander gekoppelt. Die Verbindung 37 kann beispielsweise als ziehharmonika-artig faltbares Kunststoffteil ausgebildet sein, welches eine Verschiebung in mehreren Ebenen und Richtungen der gegeneinander verschiebbaren Teile gestattet. Die Verbindung 37 schließt den zwischen den Bauteilen beim Strecken vorhandenen Spalt und verhindert so das Eindringen von Schmutz und Fremdkörpern in die darunterliegende Vorrichtung.

Obwohl in den Figuren nicht jeweils explizit dargestellt, kann eine derartige Verbindung zwischen allen Platten, vorzugsweise jeweils in Längsrichtung des Moduls 30 oder der Liege 2 angeordnet sein.

Nachfolgend wird auf Fig. 11 Bezug genommen. Diese zeigt in einer weiteren Explosionsdarstellung die Bestandteile des Moduls, welches für das in Fig. 7 bis Fig. 10 dargestellte Gerät zum Strecken des Benutzers verwendet wird, bei welchem jedoch das Strecken in drei Ebenen oder Richtungen einstellbar ist.

Durch weitere Lager 38 sind das Kopfteil mit dem Polster 32a und der Platte 36a senkrecht zu deren Längsrichtung, somit in Richtung des Pfeils Y linear verschiebbar. Mittels der Lager 39 und 40 ist auch die Platte 36b senkrecht zu deren Haupterstreckungsrichtung, somit in Richtung des Pfeils Y linear verfahrbar.

Mittels der Lager 39 und 40 ist es auch möglich, die Platte 36b um die mit dem Pfeil 40 dargestellte Rotationsrichtung zumindest in kleinen Winkelbereichen zu drehen, insbesondere durch eine nicht identische Bewegung mittels des Lagers 39 relativ zu dem Lager 40. Es liegt im Rahmen der vorliegenden Offenbarung, zusätzlich oder alternativ zu den Lagern 38 und 39 die Platte 36b auf einem rotationsfähigen Lager anzuordnen, um größere Winkel in der mit dem Pfeil 43 bezeichneten Rotationsrichtung zu gestatten.

Mit weiteren Lineartranslatoren 42 kann die Platte 36b an deren distalen Ende in Richtung des Pfeils z angehoben oder abgesenkt werden. Werden die Lineartranslatoren 42 verschieden angesteuert, kann hierdurch auch eine Drehung der Platte in der mit dem Pfeil 41 dargestellten Richtung vorgenommen werden.

Die Platte 36b kann also zumindest in drei Richtungen des Raums unabhängig verschoben werden, dies ist als erste Richtung die Richtung entlang von deren Längsachse, sowie als zweite Richtung die in Figur 11 dargestellt Richtung y, quer zu deren Längsachse horizontal verlaufend, insbesondere unter einem senkrechten Winkel von 90° horizontal verlaufend zu deren Längsachse und ist als dritte Richtung die mit dem Pfeil Z in Figur 11 dargestellte Richtung quer zu deren Längsachse vertikal verlaufend, insbesondere unter einem senkrechten Winkel von 90° vertikal verlaufend zu deren Längsachse, wobei in dieser Richtung vorzugsweise nur das distale Ende der Platte 36b angehoben wird, an welchem die Lineartranslatoren 42 angeordnet sind und hierdurch die Platte 36b an deren distalen Ende, somit deren Fußende angehoben und abgesenkt werden kann.

Darüber hinaus kann die Platte auch in zumindest zwei zueinander orthogonalen Richtungen jeweils unabhängig verdreht werden. Dies sind die mit den Pfeilen 41 und 43 bezeichneten Richtungen. Die mit dem Pfeil 41 bezeichnete Drehrichtung wird beispielsweise in der Avionik auch als Rollen oder Rollrichtung und die mit dem Pfeil 43 bezeichnete Richtung auch als Gieren oder Gier-Richtung bezeichnet.

In Summe kann die Platte somit unabhängig jeweils in drei orthogonalen Raumrichtungen verstellt, insbesondere in deren Lage definiert eingestellt werden.

Es liegt im Rahmen der vorliegenden Offenbarung, dass zwar die bevorzugteste Ausführungsform alle der vorstehend erwähnten Verstellmöglichkeiten bietet jedoch auch alle Unterkombinationen von der vorliegenden Offenbarung mit umfasst sind, welche nur eine oder einen Teil der vorstehend erwähnten Verstellmöglichkeiten aufweisen. Durch die Erfindung können insbesondere die Behandlungserfolge bei Arthrose-Patienten merklich verbessert werden.

### Bezugszeichenliste

- 1: Gerät zur Kernspinresonanztherapie
- 2: Liege
- 3: Benutzer
- 4: Kopfstütze
- 5: Seitenteil
- 6: Seitenteil
- 7: Träger
- 8: Steuereinrichtung
- 9: Gurt
- 10: Gurt
- 11: Verbindungsgurt
- 12: Zugvorrichtung
- 13: Umlenkrolle
- 14: Gurt
- 15: Gehäuse
- 16: Schlitten
- 17a-17c: Platte
- 18: Öse für einen Gurt
- 19: Antrieb
- 20: Führung
- 21: Anzeige
- 22: Schlitten
- 23: Kraftsensor
- 24: Verbinder
- 25: Stange
- 26: Schiene
- 27: Rahmen
- 28: Spindel
- 29: Verstrebung
- 30: nachrüstbares Modul
- 31: Spule
- 32a: Polster
- 32b: Polster
- 33: Skala
- 34a: Schiene
- 34b: Schiene
- 35: Lager
- 36a: Platte
- 36b: Platte
- 37: Verbindung
- 38: Lager
- 39: Lager
- 40: Lager
- 41: Pfeil zur Bezeichnung einer Rotationsrichtung
- 42: Lineartranslator
- 43: Pfeil zur Bezeichnung einer Rotationsrichtung
- 44: Lagerpunkt oder Lagerstange

- X: Pfeil zur Bezeichnung einer Richtung, insbesondere einer Richtung einer Verkippung
- Y: Pfeil zur Bezeichnung einer Richtung, insbesondere einer Bewegungsrichtung
- Z: Pfeil zur Bezeichnung einer Richtung, insbesondere einer Bewegungsrichtung

## Patentansprüche

1. Gerät zur Kernspinresonanztherapie, umfassend eine Einrichtung zur Erzeugung eines magnetischen Wechselfeldes, eine Einrichtung zur Erzeugung eines quer zum magnetischen Wechselfeld verlaufenden magnetischen Sweepfeldes, sowie eine Vorrichtung zum Strecken eines Benutzers, wobei eine periodisch wechselnde Kraft während einer Behandlung mit Kernspinresonanzen erzeugbar ist.

2. Gerät zur Kernspinresonanztherapie nach dem vorstehenden Anspruch, **dadurch gekennzeichnet, dass** die Vorrichtung zum Strecken des Benutzers Gurte zum Anlegen sowie zumindest eine Zugvorrichtung für einen Gurt umfasst.

3. Gerät zur Kernspinresonanztherapie nach einem der vorstehenden Ansprüche, **dadurch gekennzeichnet, dass** die Vorrichtung zum Strecken des Benutzers eine verschiebbare Platte umfasst, insbesondere eine als verschiebbares Mittel- oder Endteil ausgebildete Platte, wobei die Platte vorzugsweise über einen stirnseitigen Antrieb verschiebbar ist, insbesondere einen Antrieb mit einer Gewindespindel.

4. Gerät zur Kernspinresonanztherapie nach einem der beiden vorstehenden Ansprüche, **dadurch gekennzeichnet, dass** die Vorrichtung zum Strecken des Benutzers als auf ein Gehäuse aufsetzbares Modul ausgebildet ist.

5. Gerät zur Kernspinresonanztherapie nach einem der vorstehenden Ansprüche, **dadurch gekennzeichnet, dass** mittels der Vorrichtung zum Strecken des Benutzers, insbesondere über eine Zugvorrichtung, eine periodisch wechselnde Kraft erzeugbar ist, wobei die Frequenz der periodisch wechselnden Kraft vorzugsweise 5 bis 1 kHz beträgt.

6. Gerät zur Kernspinresonanztherapie nach einem der vorstehenden Ansprüche, **dadurch gekennzeichnet, dass** die Vorrichtung zum Strecken des Benutzers einen Aktuator zum Aufbringen einer Schwingung auf eine Zugvorrichtung umfasst.

7. Gerät zur Kernspinresonanztherapie nach dem vorstehenden Anspruch, **dadurch gekennzeichnet, dass** der Aktuator ausgebildet ist, mit einer Frequenz zwischen 1 und 500 Hz, bevorzugt zwischen 30 und 80 Hz, zu schwingen.

8. Gerät zur Kernspinresonanztherapie nach einem der vorstehenden Ansprüche, **dadurch gekennzeichnet, dass** das Gerät eine Steuereinrichtung für die Einrichtung zur Erzeugung des magnetischen Wechselfeldes und zur Steuerung des Sweepfeldes sowie zur Steuerung der Vorrichtung zum Strecken des Benutzers umfasst.

9. Gerät zur Kernspinresonanztherapie nach dem vorstehenden Anspruch, **dadurch gekennzeichnet, dass** mittels der Steuerung eine von einer Zugvorrichtung erzeugte Frequenz an eine Frequenz des Sweepfeldes gekoppelt ist.

10. Gerät zur Kernspinresonanztherapie nach einem der vorstehenden Ansprüche, **dadurch gekennzeichnet, dass** das Gerät zur Kernspinresonanztherapie eine Liege für den Benutzer umfasst, wobei seitlich an der Liege zwei Seitenteile mit jeweils einer Spule angeordnet sind, welche Teil der Einrichtung zur Erzeugung des Sweepfeldes sind und/oder dass die Vorrichtung zum Strecken des Benutzers eine Kraftmesseinrichtung umfasst.

11. Modul zum Strecken eines Benutzers für ein Gerät zur Kernspinresonanztherapie nach einem der vorstehenden Ansprüche, umfassend einen auf ein Gehäuse aufsetzbaren Rahmen mit zumindest zwei Platten, die eine Liegeflächen bilden, wobei eine der Platten gegenüber der anderen Platte mittels eines Antriebs verschiebbar ist.

12. Modul zum Strecken eines Benutzers nach dem vorstehenden Anspruch, **dadurch gekennzeichnet, dass** das Modul drei oder mehr als drei, insbesondere auch fünf Platten umfasst, wobei zumindest eine mittlere oder vordere oder jede der Platten verschiebbar ist, insbesondere auf Schienen, die am Rahmen angeordnet sind, insbesondere mittels eines Antriebs, der eine zumindest abschnittsweise unter einer nicht verschiebbaren Platte laufende Spindel oder Stange umfasst.

13. Modul zum Strecken eines Benutzers nach einem der beiden vorstehenden Ansprüche, bei welchem zumindest eine der Platten, bevorzugt mehr als eine der Platten, zumindest in drei Richtungen des Raums unabhängig verschiebbar ist,
wobei in einer Richtung, vorzugsweise vertikalen Richtung das distale Ende der Platte anhebbar ist, an welchem vorzugsweise Lineartranslatoren angeordnet sind und hierdurch die Platte an deren distalen Ende, somit deren Fußende anhebbar und absenkbar ist.

14. Modul zum Strecken eines Benutzers nach einem der drei vorstehenden Ansprüche, bei welchem zumindest eine der Platten, bevorzugt mehr als eine der Platten auch in zumindest zwei zueinander orthogonalen Richtungen jeweils unabhängig verdrehbar ist.

15. Modul zum Strecken eines Benutzers nach einem der vier vorstehenden Ansprüche, bei welchem die Platten, insbesondere bis zu fünf oder mehr einzelne Liegenplatten umfassen, vorzugsweise jeweils ein quer, insbesondere senkrecht zur jeweiligen Längsrichtung der Platte angeordneter Lagerpunkt oder eine Lagerstange vorgesehen, um eine Verstellung, insbesondere Verkippung der einzelnen Platte der Liege um die jeweilige Achse des Lagerpunktes mechanisch oder elektrisch angesteuert zu ermöglichen.

## Claims

1. Device for nuclear magnetic resonance therapy, comprising a device for generating an alternating magnetic field, a device for generating a magnetic field running transversely to the sweep field, as well as device for stretching a user, wherein a periodically alternating force can be generated during a treatment with magnetic resonances.

2. Device for nuclear magnetic resonance therapy according to the above claim, **characterized in that** the device for stretching the user comprises straps for use as well as at least one pulling device for a strap.

3. Device for nuclear magnetic resonance therapy according to one of the above claims, **characterized in that** the device for stretching the user comprises a movable board, particularly a board taking the form of a movable middle or end part, wherein the board preferably can be moved by a drive on the narrow end, in particular a drive with a threaded spindle.

4. Device for nuclear magnetic resonance therapy according to one of the two above claims, **characterized in that** the device for stretching the user takes the form of a module which can be placed on a casing.

5. Device for nuclear magnetic resonance therapy according to one of the above claims, **characterized in that** by means of the device for stretching the user, particularly by a pulling device, a periodically changing force can be generated, wherein the frequency of the periodically changing force is 5 to 1 kHz.

6. Device for nuclear magnetic resonance therapy according to one of the above claims, **characterized in that** the device for stretching the user comprises an actuator for generating a vibration on a pulling device.

7. Device for nuclear magnetic resonance therapy according to the above claim, **characterized in that** the actuator is designed to vibrate with a frequency of between 1 and 500 Hz, preferably between 30 and 80 Hz.

8. Device for nuclear magnetic resonance therapy according to one of the above claims, **characterized in that** the device includes a control device for the device for generating an alternating magnetic field and for controlling the sweep field as well as controlling the device for stretching the user.

9. Device for nuclear magnetic resonance therapy according to the above claim, **characterized in that** a frequency generated by a pulling device is connected to a frequency of the sweep field by means of the control device.

10. Device for nuclear magnetic resonance therapy according to one of the above claims, **characterized in that** the device for nuclear magnetic resonance therapy comprises a table for the user, wherein two side parts each with a coil are arranged at the side of the table and form part of the device for generating the sweep field and/or that the device for stretching the user includes a force measurement device.

11. Module for stretching a user for a device for nuclear magnetic resonance therapy according to one of the above claims, having a frame which can be placed on a casing and having at least two boards which form a table, wherein one of the boards can be moved in relation to the other board by means of a drive.

12. Module for stretching a user according to the above claim, **characterized in that** the module comprises three or more than three, in particular also five boards, wherein at least one middle or front board or each of the boards can be moved, in particular on rails which are arranged on the frame, in particular by a drive which comprises, at least in sections, a spindle or bar running under one of the non-movable boards.

13. Module for stretching a user according to one of the two above claims in which at least one of the boards, preferably more than one of the boards, can be moved independently at least in three spatial directions, wherein in one direction, preferably the vertical direction, the distal end of the board can be lifted, on which preferably linear translators are arranged and hereby the board can be raised and lowered at its distal end, consequently its foot end.

14. Module for stretching a user according to one of the three above claims in which at least one of the boards, preferably more than one of the boards, can be rotated also in at least two directions orthogonal to each other and independently of each other.

15. Module for stretching a user according to one of the four above claims in which the boards comprise in particular up to five or more individual boards, preferably each of them with a transverse bearing point or bearing bar arranged at right angles to the longitudinal direction of the board in order to enable a movement, in particular a tilting of the individual board of the table round the relevant axis activated mechanically or electrically.

## Revendications

1. Appareil de thérapie par résonance magnétique nucléaire, comprenant un système de production d'un champ alternatif magnétique, un système de production d'un champ de balayage magnétique s'étendant de manière transversale par rapport au champ alternatif magnétique, ainsi qu'un dispositif d'étirage d'un utilisateur, dans lequel une force alternante périodiquement peut être produite avec des résonances magnétiques nucléaires au cours d'un traitement.

2. Appareil de thérapie par résonance magnétique nucléaire selon la revendication précédente, **caractérisé en ce que** le dispositif d'étirage de l'utilisateur comprend des sangles à poser ainsi qu'au moins un dispositif de traction pour une sangle.

3. Appareil de thérapie par résonance magnétique nucléaire selon l'une quelconque des revendications précédentes, **caractérisé en ce que** le dispositif d'étirage de l'utilisateur comprend un panneau pouvant être coulissé, en particulier un panneau réalisé en tant que partie centrale ou partie d'extrémité pouvant être coulissée, dans lequel le panneau peut être coulissé de préférence par l'intermédiaire d'un entraînement côté frontal, en particulier un entraînement avec une broche filetée.

4. Appareil de thérapie par résonance magnétique nucléaire selon l'une quelconque des deux revendications précédentes, **caractérisé en ce que** le dispositif d'étirage de l'utilisateur est réalisé en tant que module pouvant être placé sur un boîtier.

5. Appareil de thérapie par résonance magnétique nucléaire selon l'une quelconque des revendications précédentes, **caractérisé en ce qu'**une force alternante périodiquement peut être produite au moyen du dispositif d'étirage de l'utilisateur, en particulier par l'intermédiaire d'un dispositif de traction, dans lequel la fréquence de la force alternante périodiquement va de préférence de 5 à 1 kHz.

6. Appareil de thérapie par résonance magnétique nucléaire selon l'une quelconque des revendications précédentes, **caractérisé en ce que** le dispositif d'étirage de l'utilisateur comprend un actionneur destiné à appliquer une vibration sur un dispositif de traction.

7. Appareil de thérapie par résonance magnétique nucléaire selon la revendication précédente, **caractérisé en ce que** l'actionneur est réalisé pour vibrer à une fréquence entre 1 et 500 Hz, de manière préférée entre 30 et 80 Hz.

8. Appareil de thérapie par résonance magnétique nucléaire selon l'une quelconque des revendications précédentes, **caractérisé en ce que** l'appareil comprend un système de commande pour le système de production du champ alternatif magnétique et de commande du champ de balayage ainsi que de commande du dispositif d'étirage de l'utilisateur.

9. Appareil de thérapie par résonance magnétique nucléaire selon la revendication précédente, **caractérisé en ce qu'**une fréquence produite par un dispositif de traction est couplée à une fréquence du champ de balayage au moyen de la commande.

10. Appareil de thérapie par résonance magnétique nucléaire selon l'une quelconque des revendications précédentes, **caractérisé en ce que** l'appareil de thérapie par résonance magnétique nucléaire comprend un couchage pour l'utilisateur, dans lequel deux parties latérales avec respectivement une bobine sont disposées à côté du couchage, lesquelles font partie du système de production du champ de balayage, et/ou que le dispositif d'étirage de l'utilisateur comprend un système de mesure de force.

11. Module d'étirage d'un utilisateur pour un appareil de traitement par résonance magnétique nucléaire selon l'une quelconque des revendications précédentes, comprenant un châssis pouvant être placé sur un boîtier avec au moins deux panneaux, qui forment une surface de couchage, dans lequel un des panneaux peut être coulissé au moyen d'un entraînement par rapport à l'autre panneau.

12. Module d'étirage d'un utilisateur selon la revendication précédente, **caractérisé en ce que** le module comprend trois ou plus de trois, en particulier également cinq panneaux, dans lequel au moins un panneau central ou un panneau avant ou chacun des panneaux peut être coulissé, en particulier sur des rails, qui sont disposés sur le châssis, en particulier au moyen d'un entraînement, qui comprend une broche ou une tige allant au moins par endroits sous un panneau ne pouvant pas être coulissé.

13. Module d'étirage d'un utilisateur selon l'une quelconque des deux revendications précédentes, où au moins un des panneaux, de manière préférée plus d'un des panneaux, peut être coulissé au moins dans trois directions de l'espace indépendamment,
ans lequel l'extrémité distale du panneau peut être soulevée dans une direction, de préférence une direction verticale, sur laquelle de préférence des équipements de translation linéaire sont disposés, et le panneau peut être relevé et abaissé ce faisant sur son extrémité distale, donc sur son extrémité de pied.

14. Module d'étirage d'un utilisateur selon l'une quelconque des trois revendications précédentes, où au moins un des panneaux, de manière préférée plus d'un des panneaux, peut être tourné respectivement indépendamment également dans au moins deux directions orthogonales l'une par rapport à l'autre.

15. Module d'étirage d'un utilisateur selon l'une quelconque des quatre revendications précédentes, où les panneaux comprennent en particulier jusqu'à cinq panneaux de couchage individuels ou plus, de préférence respectivement un point de palier ou une tige de palier disposé ou disposée de manière transversale, en particulier de manière perpendiculaire par rapport à la direction longitudinale respective du panneau est prévu ou prévue pour permettre avec un pilotage mécanique ou électrique un ajustement, en particulier un basculement du panneau individuel du couchage autour de l'axe respectif du point de palier.
